# FASCICULE DE BREVET EUROPEEN

(11) **EP 0 628 300 B1**
(45) Date de publication et mention de la délivrance du brevet: **13.10.1999**
(21) Numéro de dépôt: 94400512.3
(22) Date de dépôt: 09.03.1994
(51) Int. Cl.: A61K 7/00

(54) **Composition cosmétique colorée**
Gefärbte kosmetische Zusammensetzung
Coulored cosmetic composition

(30) Priorité: 06.04.1993 FR 9304061
(43) Date de publication de la demande: 14.12.1994
(73) Titulaire: L'OREAL, 75008 Paris (FR)
(72) Inventeur: Gueret, Jean-Louis, F-75018 Paris (FR); Arraudeau, Jean-Pierre, F-75015 Paris (FR)
(74) Mandataire: Peuscet, Jacques

(56) Documents cités:
- EP-A- 0 279 319
- EP-A- 0 504 066
- WO-A-91/06277
- FR-A- 2 563 104
- FR-A- 2 651 789
- FR-A- 2 664 160
- GB-A- 1 169 323
- US-A- 4 919 922

## Description

La présente invention concerne une composition cosmétique colorée contenant une charge particulaire.

Il est connu de préparer des compositions cosmétiques colorées contenant des charges particulaires solides. Ces compositions cosmétiques sont notamment des rouges à lèvres, des poudres libres ou compactées, des fonds de teint sous forme de crème, des rouges à joues ou blush, des eye liners, des fards à paupières, des mascaras ou des vernis à ongles.

Dans les compositions ci-dessus, les charges et les colorants sont souvent introduits séparément et, dans ce cas, les colorants sont libres, ce qui peut présenter un inconvénient sur le plan de l'innocuité de la composition ; certains colorants, qui donneraient une couleur particulièrement attrayante, ne peuvent notamment pas être utilisés car ils présentent une toxicité notable dans les conditions de mise en oeuvre de la composition : par exemple, le vert de phtalocyanine chloré (codifié dans le Color Index sous le n° 74 260) ne peut pas être utilisé pour la fabrication de compositions cosmétiques, qui risquent d'être en contact avec la muqueuse des yeux, car il est toxique dans ces conditions. De plus, pour certains colorants, il est nécessaire, en vue de leur utilisation dans des compositions cosmétiques, de surveiller leur pureté avec beaucoup de précaution pour éviter tout risque de toxicité par des impuretés du colorant.

On a déjà proposé de teinter des charges dans la masse pour éviter d'avoir dans la composition des colorants libres ; néanmoins le colorant peut être partiellement relargué par la charge dans les conditions d'application et, en outre, pour obtenir une coloration suffisamment intense, il est nécessaire d'introduire une quantité de colorants relativement élevée puisqu'il faut teinter tout le volume des particules de charge d'où il résulte un accroissement du coût de la composition.

Selon la présente invention, on a trouvé que l'on peut introduire dans des compositions cosmétiques des colorants, même considérés généralement comme toxiques, en n'introduisant plus séparément dans la composition cosmétique la charge particulaire et le colorant, mais en introduisant au moins une partie de la charge sous forme de particules enrobées par un polymère contenant au moins un colorant, la totalité des colorants étant associée au polymère enrobant la charge.

La présente invention a, par conséquent, pour objet une composition cosmétique macroscopiquement colorée contenant au moins une charge particulaire et au moins un colorant, caractérisée par le fait qu'au moins une partie des charges de la composition est enrobée par un polymère associé à au moins un colorant.

On préfère que la totalité du (ou des) colorant(s) de la composition soit associée à au moins un polymère enrobant une charge particulaire. Les particules de la charge enrobée ont, de préférence, des dimensions comprises entre 1 et 200 µm dans leur plus petite dimension.

Selon la présente invention, la charge est, de préférence, enrobée de polymère associé à au moins un colorant par le procédé décrit dans FR-A 2 651 789. Selon ce procédé, on enrobe la charge pulvérulente à l'aide d'une solution de l'un au moins des constituants du polymère fini, on élimine le solvant puis on fait réagir sur la charge pulvérulente enrobée un autre constituant du polymère. Le polymère est ainsi préparé in situ. Le colorant est introduit sous forme de suspension ou de solution dans la solution d'enrobage de la charge. Dans la présente description, on appelera, par la suite, polymère coloré un polymère contenant au moins un colorant répartie dans sa masse sous forme de solution ou de suspension.

Selon FR-A 2 651 789, le solvant est un solvant organique, mais on a trouvé que le solvant pouvait également, dans certains cas, être une phase aqueuse.

Comme expliqué ci-dessus, l'utilisation dans une composition cosmétique d'une charge enrobée de polymère coloré permet l'utilisation de colorants relativement toxiques et permet d'utiliser une plus faible quantité de colorant pour obtenir une coloration donnée puisque tout le colorant se trouve dans l'enrobage des particules de charge, donc à la surface desdites particules, et non plus réparti dans tout le volume des particules, ce qui était le cas pour les charges teintées de l'état de la technique. De plus, la mise en place du colorant dans le film d'enrobage polymérique permet d'améliorer la stabilité à la lumière du colorant ; par exemple, on a constaté que le violet de manganèse (codifié dans le Color Index (CI) sous la référence 77 742) peut être utilisé de la sorte, sans que la couleur de la composition cosmétique pâlisse à la lumière. Un avantage supplémentaire est que la présence du polymère enrobant la charge permet de modifier sa densité, ce qui peut notamment éviter des sédimentations ; en outre, l'enrobage peut aussi modifier l'état de surface des particules : la charge peut donc ainsi devenir hydrophobe ou hydrophile après enrobage. L'épaisseur du film d'enrobage polymérique est généralement comprise entre 0,2 et 50 µm.

La(les) charge(s) enrobée(s), selon l'invention, peu(ven)t être toute charge utilisable dans le domaine cosmétique, qu'elle soit minérale ou organique ; avant enrobage, une telle charge a généralement une granulométrie comprise entre 0,8 et 180 µm. Parmi les charges minérales, on peut citer le plâtre, le carbonate de calcium, le mica, la silice, le silicate de calcium, le kaolin, les billes de verre, l'oxyde de titane, l'oxyde de zinc, l'oxyde de zirconium et l'alumine. Parmi les charges organiques, on peut citer les fibres de cellulose, par exemple fibres de coton ou poudre de bois, l'amidon, des microsphères expansées, par exemple de copolymère polyvinylidène/acrylonitrile, des poudres de polyéthylène, de nylon, de silicone, de polypropylène, de polycarbonate, de résine urée-formol, de gélatine réticulée, de collagène, de kératine, de polystyrène et de téflon. La charge peut être également constituée par tout mélange de charges minérales et/ou organiques.

Dans la composition selon l'invention, la charge enrobée colorée peut être mélangée à au moins une charge non colorée et/ou non enrobée. La composition peut aussi contenir plusieurs charges, identiques ou différentes, enrobées par des polymères différents associés à des colorants identiques ou différents.

Le polymère préparé in situ sur les charges peut être tout polymère de polyaddition dans lequel un des constituants peut être mis en solution en présence d'un colorant pour enrober la charge et l'autre constituant peut être mis en contact avec la charge enrobée pour former le polymère ; ce peut être aussi un polymère réticulable pouvant être mis en solution en présence d'un colorant pour enrober la charge, l'autre constituant étant alors un agent de réticulation.

Parmi les polymères préparables sur la charge, on peut citer les polyuréthannes, les résines époxy, les polyéthers chlorés, les polyesters, les urée-formols, les homo- ou copolymères acryliques, methacryliques et vinyliques.

Selon un mode de préparation préféré, on enrobe la charge à l'aide d'une solution d'uréthanne ou de silicone aminofonctionnel comme polymère réticulable et on utilise comme agent de réticulation un composé choisi parmi les isocyanates.

Les colorants utilisés peuvent être, bien entendu, tous les colorants utilisables en cosmétique, qu'ils soient des pigments minéraux ou des colorants organiques. Les colorants sont utilisés soit sous forme de solution, soit sous forme de dispersion de particules solides ayant une granulométrie comprise entre 0,1 et 25 µm, auquel cas le diamètre des particules de colorant est inférieur à celui des particules de charges avant enrobage.

Parmi les pigments minéraux généralement utilisés en cosmétique, on peut citer :
- le dioxyde de titane (rutile ou anatase) éventuellement traité en surface (codifié dans le Color Index sous la référence CI 77 891),
- le violet de manganèse (CI 77 742),
- le bleu ultramarine (CI 77 007),
- l'oxyde de chrome (CI 77 288),
- l'oxyde de chrome hydraté (CI 77 289),
- le bleu ferrique (CI 77 510),
- l'oxyde de zinc,
- le dioxyde de zirconium.

Parmi les colorants organiques généralement utilisés en cosmétique, on peut citer :
- D & C red n° 19 (CI 45 170),
- D & C red n° 9 (CI 15 585),
- D & C red n° 21 (CI 45 380),
- D & C orange n° 4 (CI 15 510),
- D & C orange n° 5 (CI 45 370),
- D & C red n° 27 (CI 45 410),
- D & C red n° 13 (CI 15 630),
- D & C red n° 7 (CI 15 850 : 1),
- D & C red n° 6 (CI 15 850 : 2),
- D & C yellow n°5 (CI 19 140),
- D & C red n° 36 (CI 12 085),
- D & C orange n° 10 (CI 45 425),
- D & C yellow n° 6 (CI 15 985),
- D & C red n° 30 (CI 73 360),
- D & C red n° 3 (CI 45 430),
- le noir de carbone (CI 77 266),
- la laque carmin de cochenille (CI 75 470),
- la mélanine naturelle ou synthétique et
- les laques d'aluminium.

Mais il est également possible, selon l'invention, d'utiliser sans risque de toxicité des colorants, qui sont considérés comme nocifs pour la muqueuse des yeux et/ou pour la peau et ne sont, par conséquent, généralement pas utilisés jusqu'à présent dans des compositions cosmétiques.

On peut, selon l'invention, utiliser un ou plusieurs colorants minéraux et/ou organiques soit associés à une même charge, soit à des charges différentes.

Les exemples donnés ci-après, à titre purement illustratif et non limitatif, permettront de mieux comprendre l'invention.

### EXEMPLE 1 : Poudre compactée

Une première charge enrobée colorée est constituée par du talc d'une granulométrie moyenne de 10 µm enrobé d'un film de polyuréthanne ayant une épaisseur d'environ 2 µm et contenant 2 % en poids de bleu ultramarine (CI 77 007) d'une granulométrie de 0,2 µm environ. Une deuxième charge enrobée colorée est constituée par du mica d'une granulométrie moyenne de 10 µm enrobé d'un film de résine époxy d'environ 3 µm d'épaisseur et contenant du bleu ferrique (CI 77 510) ayant une granulométrie d'environ 0,5 µm.

On prépare un mélange contenant (en % en poids) :

| | |
|---|---|
| - 1ère charge enrobée colorée ci-dessus définie | 40 % |
| - 2ème charge enrobée colorée ci-dessus définie | 20 % |
| - Dioxyde de titane | 20 % |
| - Mica | 5 % |
| - Myristate d'isopropyle | 2 % |
| - Huile de vaseline | 2 % |
| - Microsphères de polyacrylate expansé vendues sous la dénomination commerciale "EXPANCEL 551 DE 20" par la société "CASCO-NOBEL" | 0,5 % |
| - Sorbitol | 0,5 % |
| - Talc | 10 % |

On compacte le mélange. On peut appliquer cette poudre compactée de couleur bleue chinée à l'aide d'une houppette ou d'un pinceau. On constate que cette poudre présente une stabilité de nuance remarquable.

### EXEMPLE 2 : Poudre libre à appliquer avec une houppette

On utilise un mélange ayant la composition suivante :

| | |
|---|---|
| - Charge constituée par de la poudre de nylon vendue sous la dénomination commerciale "ORGASOL 2002 D" par la société "ATOCHEM" (granulométrie d'environ 10 µm) enrobée d'une couche de 2 µm de copolymère acrylique contenant 2 % en poids de colorants constitués par des oxydes de fer jaune et noir et du D & C Red 30 dans les proportions 4/2/1 (granulométrie d'environ 0,5 µm) | 37,75 % |
| - Mica | 55,45 % |
| - Microsphères creuses de copolymère de type chlorure de vinylidène-acrylonitrile commercialisées sous la dénomination "EXPANCEL 551 DE" par la société "CASCO NOBEL" | 1 % |
| - Myristate de magnésium | 2 % |
| - Huile de vaseline | 3 % |
| - Parfum | 0,8 % |

La poudre est préparée de la façon suivante : le mica, la charge enrobée et le myristate de magnésium sont mélangés, puis broyés. Le broyat est ensuite pulvérisé avec le parfum, puis on ajoute l'huile de vaseline. L'"EXPANCEL 551 DE" est finalement additionné par simple mélange. On obtient une poudre libre de couleur pêche dorée.

### EXEMPLE 3 : Vernis à ongles

On utilise une charge constituée par une poudre de CaCO₃ de granulométrie voisine de 12 µm préalablement enrobée d'une résine époxy constituant une couche d'environ 3 µm dans laquelle sont emprisonnés à raison de 4 % en poids des pigments ayant une granulométrie de 2 à 5 µm dans les proportions suivantes (en poids) : TiO₂ = 100, oxyde de fer noir = 10, oxyde de fer rouge = 5, D & C Red n° 6 (laque de Ba) = 10.

Le vernis à ongles préparé a la composition suivante (en % en poids) :

| | |
|---|---|
| - n-butyl acétate | 25 % |
| - Toluène | 25 % |
| - Ethyl acétate | 10 % |
| - Alcool d'isopropyl | 6,75 % |
| - Bentone 27 (montmorillonite) | 1 % |
| - Nitrocellulose | 16 % |
| - Résine de toluène sulfonamide formaldéhyde | 9 % |
| - Dibutyl phthalate | 5 % |
| - Camphre | 1 % |
| - Charge enrobée colorée ci-dessus définie | 1,25 % |

On obtient un vernis à ongles de couleur vieux rose ayant une sédimentation non irréversible, possèdant une bonne stabilité de couleur et une non migration des colorants sur les ongles.

En outre, cette composition a l'avantage de ne pas colorer l'ongle quand l'utilisateur enlève le vernis à l'aide d'un dissolvant.

## Revendications

1. Composition cosmétique colorée contenant au moins une charge particulaire et au moins un colorant, caractérisée par le fait qu'au moins une partie des charges de la composition est enrobée par un polymère associé à au moins un colorant.

2. Composition selon la revendication 1, caractérisée par le fait que la totalité du (ou des) colorant(s) de la composition est associée à au moins un polymère enrobant une charge particulaire.

3. Composition selon l'une des revendications 1 ou 2, caractérisée par le fait que les particules de charge enrobées ont des dimensions comprises entre 1 et 200 µm dans leur plus petite dimension.

4. Composition selon l'une des revendications 1 à 3, caractérisée par le fait que la (les) charge(s) est (sont) choisie(s) dans le groupe formé par le plâtre, le carbonate de calcium, le mica, la silice, le silicate de calcium, le kaolin, les billes de verre, l'oxyde de titane, l'oxyde de zinc, l'oxyde de zirconium et l'alumine, les fibres de cellulose, l'amidon, des microsphères expansées, des poudres de polyéthylène, de nylon, de silicone, de polypropylène, de polycarbonate, de résine urée-formol, de gélatine réticulée, de collagène, de kératine, de polystyrène et de téflon, ainsi que par tout mélange de charges minérales et/ou organiques.

5. Composition selon l'une des revendications 1 à 4, caractérisée par le fait que la composition contient plusieurs charges identiques ou différentes enrobées par des polymères différents associés à des colorants identiques ou différents.

6. Composition selon l'une des revendications 1 à 5, caractérisée par le fait que le(s) colorant(s) associé(s) au polymère est (sont) sous forme de solution ou de dispersion de particules solides ayant une granulométrie comprise entre 0,1 et 25 µm environ.

7. Composition selon l'une des revendications 1 à 6, caractérisée par le fait que le polymère enrobant la (les) charge(s) est choisi dans le groupe formé par les polyuréthannes, les résines époxy, les polyéthers chlorés, les polyesters, les urée-formols, les homo- ou copolymères acryliques, methacryliques et vinyliques.

8. Composition selon l'une des revendications 1 à 7, caractérisée par le fait que le colorant est choisi dans le groupe formé par les pigments minéraux et les colorants organiques.

9. Composition selon l'une des revendications 1 à 8, caractérisée par le fait que l'épaisseur du film d'enrobage polymérique est généralement comprise entre 0,2 et 50 µm.

## Claims

1. Coloured cosmetic composition containing at least one particulate filler and at least one colorant, characterized in that at least part of the fillers of the composition is coated with a polymer combined with at least one colorant.

2. Compositon according to Claim 1, characterized in that the full complement of the colorant(s) of the composition is combined with at least one polymer coating a particulate filler.

3. Composition according to one of Claims 1 and 2, characterized in that the coated filler particles are between 1 and 200 µm in size in their smallest dimension.

4. Composition according to one of Claims 1 to 3, characterized in that the filler(s) is/are chosen from the group composed of plaster, calcium carbonate, mica, silica, calcium silicate, kaolin, glass beads, titanium oxide, zinc oxide, zirconium oxide and alumina, cellulose fibres, starch, expanded microspheres, powdered polyethylene, powdered nylon, powdered silicone, powdered polypropylene, powdered polycarbonate, powdered urea-formaldehyde resin, powdered crosslinked gelatin, powdered collagen, powdered keratin, powdered polystyrene and powdered Teflon, as well as of any mixture of inorganic and/or organic fillers.

5. Composition according to one of Claims 1 to 4, characterized in that the composition contains several identical or different fillers coated with different polymers combined with identical or different colorants.

6. Composition according to one of Claims 1 to 5, characterized in that the colorant(s) combined with the polymer is/are in solution form or the form of a dispersion of solid particles having a particle size of between 0.1 and 25 µm approximately.

7. Composition according to one of Claims 1 to 6, characterized in that the polymer coating the filler(s) is chosen from the group composed of polyurethanes, epoxy resins, chlorinated polyethers, polyesters, urea-formaldehyde resins and acrylic, methacrylic and vinyl homo- or copolymers.

8. Composition according to one of Claims 1 to 7, characterized in that the colorant is chosen from the group composed of inorganic pigments and organic colorants.

9. Composition according to one of Claims 1 to 8, characterized in that the thickness of the film of polymer coating is generally between 0.2 and 50 µm.

## Patentansprüche

1. Gefärbte kosmetische Zusammensetzung, die mindestens einen Teilchenfüllstoff und mindestens einen Farbstoff enthält, dadurch gekennzeichnet, daß mindestens ein Teil der Füllstoffe der Zusammensetzung mit einem Polymer umhüllt ist, das mit mindestens einem Farbstoff kombiniert ist.

2. Zusammensetzung nach Anspruch 1, dadurch gekennzeichnet, daß die Gesamtheit des oder der Farbstoffe der Zusammensetzung mit mindestens einem einen Teilchenfüllstoff umhüllenden Polymer kombiniert ist.

3. Zusammensetzung nach einem der Ansprüche 1 oder 2, dadurch gekennzeichnet, daß die umhüllten Füllstoffteilchen in ihrer kleinsten Größe Abmessungen zwischen 1 und 200 µm haben.

4. Zusammensetzung nach einem der Ansprüche 1 bis 3, dadurch gekennzeichnet, daß der oder die Füllstoffe aus der Gruppe ausgewählt sind, die gebildet wird von Gips, Calciumcarbonat, Glimmer, Siliziumdioxid, Calciumsilicat, Kaolin, Glaskugeln, Titanoxid, Zinkoxid, Zirconiumoxid und Tonerde, Cellulosefasern, Stärke, expandierten Mikrokugeln, Pulver aus Polyethylen, Nylon, Silicon, Polypropylen, Polycarbonat, Harnstoff-Formaldehyd-Harz, vernetzter Gelatine, Collagen, Keratin, Polystyrol und Teflon, sowie von jeder Mischung von mineralischen und/oder organischen Füllstoffen.

5. Zusammensetzung nach einem der Ansprüche 1 bis 4, dadurch gekennzeichnet, daß die Zusammensetzung mehrere gleiche oder verschiedene Füllstoffe enthält, die mit verschiedenen Polymeren umhüllt sind, die mit gleichen oder verschiedenen Füllstoffen kombiniert sind.

6. Zusammensetzung nach einem der Ansprüche 1 bis 5, dadurch gekennzeichnet, daß der oder die mit dem Polymer kombinierten Farbstoffe in Form von Lösung oder Dispersion von festen Teilchen mit einer Korngröße zwischen etwa 0,1 und 25 µm vorliegen.

7. Zusammensetzung nach einem der Ansprüche 1 bis 6, dadurch gekennzeichnet, daß das den oder die Füllstoffe umhüllende Polymer aus der Gruppe ausgewählt ist, die gebildet wird von den Polyurethanen, den Epoxyharzen, den chlorierten Polyethern, den Polyestern, den Harnstoff-Formaldehyden, den Acryl-, Methacryl- und Vinyl-Homo- oder Copolymeren.

8. Zusammensetzung nach einem der Ansprüche 1 bis 7, dadurch gekennzeichnet, daß der Farbstoff aus der Gruppe ausgewählt ist, die von den mineralischen Pigmenten und organischen Farbstoffen gebildet ist.

9. Zusammensetzung nach einem der Ansprüche 1 bis 8, dadurch gekennzeichnet, daß die Dicke des Umhüllungspolymerfilms im allgemeinen zwischen 0,2 und 50 µm liegt.
